# EUROPEAN PATENT APPLICATION

(11) **EP 4 556 024 A1**
(43) Date of publication of application: **21.05.2025**
(21) Application number: 24211236.5
(22) Date of filing: 06.11.2024
(51) Int. Cl.: A61K 41/00, A61F 13/0246, A61L 24/00, A61N 5/06, A61F 13/00

(54) **LIGHT-GUIDING ADHESIVE PATCH**

(30) Priority: 06.11.2023 KR 20230151355
(71) Applicant: Samsung Life Public Welfare Foundation, Seoul 04348 (KR); Research & Business Foundation Sungkyunkwan University, Jangan-gu Suwon-si, Gyeonggi-do 16419 (KR)
(72) Inventor: CHONG, Kyu Ha, 06351 Seoul (KR); SHIN, Mi Kyung, 16419 Gyeonggi-do (KR); KIM, Jung Woo, 16419 Gyeonggi-do (KR)
(74) Representative: Weickmann & Weickmann PartmbB

(57) **Abstract**

The present disclosure relates to a light-guiding adhesive patch capable of stably adhering to the organic matter surface and effectively transmitting light to the site of attachment.

## Description

### BACKGROUND

### 1. Technical Field

The present disclosure relates to a light-guiding adhesive patch.

### 2. Related Art

Recently, photomedicine has attracted attention as a branch of medicine that deals with the application of light with respect to health and disease. It is used to image or treat diseases, and is utilized in surgery, cardiology, radiology, oncology, diagnosis, drug delivery, dermatology, ophthalmology, etc.

Although the number of cancers that can be cured is increasing due to the development of diagnostic and therapeutic technologies, cancer is still the number one cause of death. Approximately 9% to 17% of cancer patients have brain metastases, and the average survival period of patients with metastatic brain tumors is 3 to 25 months, with a 5-year survival rate of 1.8%. Melanoma, breast cancer, and lung cancer have been identified as cancers that frequently cause brain metastases, and various chemotherapy-radiotherapy regimens have been attempted. However, metastatic brain tumors, which account for approximately 50% of malignant brain tumor patients, are regarded as incurable diseases.

Recently, photodynamic therapy (PDT) has attracted attention as a new alternative therapeutic method for overcoming intractable cancer and malignant brain tumors. Photodynamic therapy has advantages, including physical selectivity in treatment through spatial and wavelength selectivity, as well as lesion selectivity that is achieved using the drug itself and a photosensitizer that responds to light of a specific wavelength, and thus it has been actively studied as an effective next-generation cancer treatment technology.

Meanwhile, in order to improve the efficiency of photodynamic therapy, a technology capable of stably transmitting light to living tissues, including cancer, is needed.

### SUMMARY

The present disclosure is intended to provide a light-guiding adhesive patch capable of stably adhering to an organic matter surface and effectively transmitting light to the site of attachment.

However, the problems to be solved by the present disclosure are not limited to the problems mentioned above, and other problems not mentioned above will be clearly understood by those skilled in the art from the following description.

One embodiment of the present disclosure provides a light-guiding adhesive patch including: an adhesive patch portion including an adhesive portion including an adhesive layer and a core layer, and a light guide layer disposed on the adhesive portion and configured to guide light to the adhesive portion; and a light-transmitting portion connected to the adhesive patch portion and configured to guide light incident from the outside to the adhesive patch portion.

The light-guiding adhesive patch according to one embodiment of the present disclosure has an advantage in that it has strong adhesiveness to the organic matter surfaces, such as *ex vivo* tissues, *in vivo* tissues, and cut tissues, and thus is capable of stably adhering to the surfaces.

In addition, the light-guiding adhesive patch according to one embodiment of the present disclosure is capable of effectively transmitting light to the site of attachment.

However, the effects of the present disclosure are not limited to the effects described above, and may be expanded in various ways without departing from the spirit and scope of the present disclosure.

### BRIEF DESCRIPTION OF THE DRAWINGS

FIG. 1a and FIG. 1b schematically illustrate light-guiding adhesive patches according to one embodiment of the present disclosure.
FIG. 2 shows the results of measuring the mechanical properties of light-transmitting cores produced in Example 1 of the present disclosure.
FIG. 3 depicts result of measuring the adhesive force of the adhesive layer prepared in Example 1 of the present invention. Specifically, the adhesive force (kPa) of the adhesive layer prepared in Example 1 is shown.
FIG. 4 depicts photographs showing that a light-transmitting portion produced in Example 1 of the present disclosure transmits light.
FIG. 5 depicts photographs showing that a light-transmitting portion produced in Example 2 of the present disclosure transmits light.
FIG. 6 is a photograph of a light-guiding adhesive patch produced in Example 1 of the present disclosure.
FIG. 7 depicts photographs showing that the light-guiding adhesive patch produced in Example 1 of the present disclosure transmits light.
FIG. 8 is a photograph of a light-guiding adhesive patch produced in Example 2 of the present disclosure.
FIG. 9 depicts photographs showing that the light-guiding adhesive patch produced in Example 2 of the present disclosure transmits light.
FIG. 10 shows a result of confirming a change in light transfer rate depending on whether an antioxidant is added to the adhesive layers of Examples 1 and 2 of the present invention.
FIG. 11 shows the results of confirming the photodynamic therapy effect of the light guide adhesive patch of the present invention. FIGS. 11A and 11C show the cell viability according to the combination of the photosensitizer and the light irradiation, and FIG. 11B shows the cell viability according to the addition of the antioxidant in the adhesive layer.
FIG. 12 shows the results of confirming the photodynamic therapy effect of the light guide adhesive patch of the present invention using 3D spheroid.

### DETAILED DESCRIPTION

Throughout the present specification, it is to be understood that when any portion is referred to as "including" any component, it does not exclude other components, but may further include other components, unless otherwise specified.

Throughout the present specification, when any member is referred to as being "connected" to another member, it not only refers to a case where any member is connected directly to the other member, but also a case where a third member exists between the two members.

Throughout the present specification, when any member is referred to as being "on" another member, it not only refers to a case where any member is in contact with another member, but also a case where a third member exists between the two members.

Throughout the present specification, the term "(meth)acrylate" is meant to include acrylate and methacrylate.

Hereinafter, the present disclosure will be described in more detail.

One embodiment of the present disclosure provides a light-guiding adhesive patch including: an adhesive patch portion including an adhesive portion including an adhesive layer and a core layer, and a light guide layer disposed on the adhesive portion and configured to guide light to the adhesive portion; and a light-transmitting portion connected to the adhesive patch portion and configured to guide light incident from the outside to the adhesive patch portion.

The light-guiding adhesive patch according to one embodiment of the present disclosure has an advantage in that it has strong adhesiveness to the organic matter surfaces, such as *ex vivo* tissues, *in vivo* tissues, and cut tissues, and thus is capable of stably adhering to the surfaces. In addition, the light-guiding adhesive patch is capable of effectively transmitting light to the site of attachment.

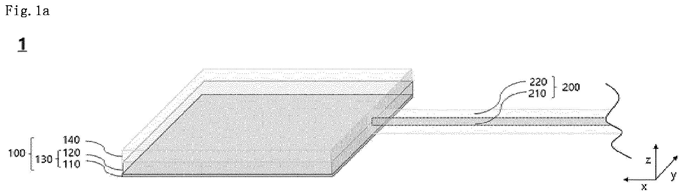

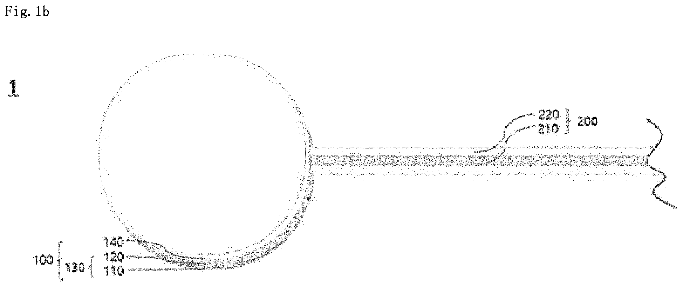

FIG. 1a and FIG. 1b schematically illustrate a light-guiding adhesive patch according to one embodiment of the present disclosure. Specifically, FIG. 1a illustrates a light-guiding adhesive patch 1 including an adhesive patch portion 100 having a rectangular cross-sectional shape, and FIG. 1b illustrates a light-guiding adhesive patch 1 including an adhesive patch portion 100 having a circular cross-sectional shape.

Referring to FIG. 1a and FIG. 1b, the light-guiding adhesive patch 1 includes an adhesive patch portion 100 that adheres to the site of attachment and transmits light to the site of attachment. In addition, the light-guiding adhesive patch 1 includes a light-transmitting portion 200 whose one end is connected to an external light source and whose other end is connected to the adhesive patch portion 100 so that the light-transmitting portion 200 transmits light incident from the light source to the adhesive patch portion 100. Here, the adhesive patch portion 100 may include an adhesive portion 130 that comes into contact with the site of attachment, and a light guide layer 140 disposed on the adhesive portion 130 and configured to guide light incident from the light-transmitting portion 200 to the adhesive portion 130.

According to one embodiment of the present disclosure, the adhesive portion may include an adhesive layer and a core layer disposed on one surface of the adhesive layer. Specifically, one surface of the adhesive layer may be a layer that comes into direct contact with the site of attachment, and the core layer may be disposed on the other surface of the adhesive layer.

According to one embodiment of the present disclosure, the adhesive layer may be a hydrogel adhesive layer including a reaction product of a biocompatible polymer and a pyrogallol-based compound. Specifically, the reaction product of the biocompatible polymer and the pyrogallol-based compound may be a hydrogel precursor. The adhesive layer including the hydrogel precursor may effectively maintain its adhesive ability even when moisture exists on the surface of an adherend.

According to one embodiment of the present disclosure, the biocompatible polymer may include at least one of polyethylene glycol, gelatin, alginate, hyaluronic acid, hyaluronic acid-adipic acid dihydrazide, polyethylene glycol amine, 4-arm polyethylene glycol amine, 6-arm polyethylene glycol amine, 8-arm polyethylene glycol amine, chitosan, and collagen. As used herein, the term "4-arm polyethylene glycol amine" may refer to a compound in which four polyethylene glycol chains having amine groups at the terminals are bonded to a main chain having a structure containing a polyhydroxy group. In addition, the term "6-arm polyethylene glycol amine" may refer to a compound in which six polyethylene glycol chains having amine groups at the terminals are bonded to a main chain having a structure containing a polyhydroxy group. In addition, the term "8-arm polyethylene glycol amine" may refer to a compound in which eight polyethylene glycol chains having amine groups at the terminals are bonded to a main chain having a structure containing a polyhydroxy group. By using the above-described type of biocompatible polymer, it is possible to effectively reduce the harmfulness of the adhesive layer to a living body when adhered to an adherend.

According to one embodiment of the present disclosure, the biocompatible polymer may include an amine group-containing biocompatible polymer and an amine group-free biocompatible polymer. For example, the amine group-containing biocompatible polymer may include at least one of polyethylene glycol amine, 4-arm polyethylene glycol amine, 6-arm polyethylene glycol amine, 8-arm polyethylene glycol amine, chitosan, and collagen. In addition, the amine group-free biocompatible polymer may include at least one of polyethylene glycol and gelatin. The amine group-containing biocompatible polymer is capable of reacting with the pyrogallol-based compound to form a compound with excellent adhesive properties. Thereby, the adhesive layer may effectively maintain its adhesive ability even when water exists on the surface of an adherend.

According to one embodiment of the present disclosure, the pyrogallol-based compound may be extracted from a plant. By using the pyrogallol-based compound extracted from a plant, it is possible to effectively impart adhesive performance to the light-guiding adhesive patch.

According to one embodiment of the present disclosure, the pyrogallol-based compound may be a compound represented by Formula 1 below. 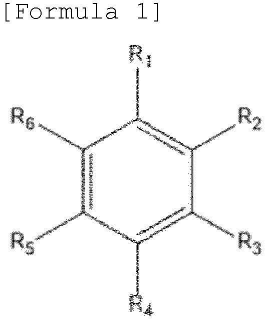 wherein R₁ is -COOH, -CHO, -NH₂, -SH, a straight or branched chain alkyl having 1 to 10 carbon atoms, or a straight or branched chain alkenyl having 2 to 10 carbon atoms; and three of R₂ to R₆ are -OH and the remaining two are hydrogen atoms. Specifically, R₁ in Formula 1 above may be -COOH, -CHO, -NH₂ or -SH. More specifically, R₁ in Formula 1 above may be -CHO. The pyrogallol-based compound represented by Formula 1 is capable of reacting with the biocompatible polymer to form a hydrogel adhesive layer having excellent adhesive properties and light-guiding properties. Specifically, the pyrogallol-based compound represented by Formula 1 is capable of reacting with the amine group-containing biocompatible polymer to form a hydrogel adhesive layer having excellent adhesive properties and light-guiding properties. Here, R₁ in Formula 1 above may be a functional group that reacts with the amine group of the amine group-containing biocompatible polymer described below.

According to one embodiment of the present disclosure, the pyrogallol-based compound may include at least one of 2,3,4-trihydroxybenzaldehyde, 2,4,5-trihydroxybenzaldehyde, 3,4,5-trihydroxybenzaldehyde, and 2,4,6-trihydroxybenzaldehyde. The pyrogallol-based compound is capable of reacting with the biocompatible polymer to form an adhesive layer having excellent adhesive properties and light-guiding properties. Specifically, the pyrogallol-based compound is capable of reacting with the amine group-containing biocompatible polymer to form a hydrogel adhesive layer having excellent adhesive and light-guiding properties.

According to one embodiment of the present disclosure, the adhesive layer may be produced using a composition for producing an adhesive layer. That is, the adhesive layer may include a reaction product of the composition for producing an adhesive layer. The composition for producing an adhesive layer may contain the biocompatible polymer and the pyrogallol-based compound. Specifically, the composition for producing an adhesive layer may contain the amine group-containing biocompatible polymer, the amine group-free biocompatible polymer, and the pyrogallol-based compound.

According to one embodiment of the present disclosure, the content of the biocompatible polymer may be 1 part by weight to 30 parts by weight based on 100 parts by weight of the composition for producing an adhesive layer. When the content of the biocompatible polymer is within the above-described range, it is possible to effectively reduce the harmfulness of the adhesive layer to a living body when adhered to an adherend, and the adhesive layer may effectively maintain its adhesive ability even when water exists on the surface of an adherend.

According to one embodiment of the present disclosure, based on 100 parts by weight of the composition for producing an adhesive layer, the content of the amine group-containing biocompatible polymer may be 0.5 parts by weight to 15 parts by weight, and the content of the amine group-free biocompatible polymer may be 0 parts by weight to 15 parts by weight. When the content of the amine group-containing biocompatible polymer and the content of the amine group-free biocompatible polymer are within the above-described ranges, it is possible to effectively reduce the harmfulness of the adhesive layer to a living body when adhered to an adherend, and the adhesive layer may effectively maintain its adhesive ability even when water exists on the surface of an adherend.

According to one embodiment of the present disclosure, the ratio of the content of the amine group-free biocompatible polymer to the content of the amine group-containing biocompatible polymer may be 1:0.5 to 1:5. Specifically, the content of the amine group-containing biocompatible polymer may be greater than the content of the amine group-free biocompatible polymer. When the ratio of the content of the amine group-free biocompatible polymer to the content of the amine group-containing biocompatible polymer is within the above-described range, it is possible to effectively reduce the harmfulness of the adhesive layer to a living body when adhered to an adherend, and the adhesive layer may effectively maintain its adhesive ability even when water exists on the surface of an adherend.

According to one embodiment of the present disclosure, the reaction product may contain the pyrogallol-based compound in an amount of 0.3 parts by weight to 30 parts by weight based on 100 parts by weight of the biocompatible polymer. That is, based on 100 parts by weight of the total content of the amine group-containing biocompatible polymer and the amine group-free biocompatible polymer contained in the composition for producing an adhesive layer, the content of the pyrogallol-based compound may be 0.3 parts by weight to 30 parts by weight. Meanwhile, the content of the pyrogallol-based compound may be an amount capable of reacting with all of the amine groups contained in the amine group-containing biocompatible polymer. Specifically, the content of the pyrogallol-based compound represented by Formula 1 may be set so that R₁ of the pyrogallol-based compound represented by Formula 1 and the amine groups contained in the amine group-containing biocompatible polymer may react at a ratio of 1:1. When the contents of the biocompatible polymer and the pyrogallol-based compound for forming the reaction product are within the above-described ranges, the adhesive layer may have strong adhesiveness to the organic matter surface, and thus stably adhere to the organic matter surface. In addition, by controlling the contents of the biocompatible polymer and the pyrogallol-based compound within the above-described ranges, it is possible to effectively improve the light-guiding properties of the adhesive layer. That is, the adhesive layer may stably adhere to the organic matter surface and effectively transmit light to the organic matter surface.

According to one embodiment of the present disclosure, the adhesive layer may further include an antioxidant. As the adhesive layer further includes the antioxidant, the light-guiding efficiency of the adhesive layer may be effectively prevented from being reduced. The antioxidant may be any antioxidant used in the art, and for example, vitamin B, vitamin C or the like may be used.

According to one embodiment of the present disclosure, the core layer may be a hydrogel including a cured product of a composition containing a photoreactive group-containing compound and a photoinitiator. The core layer may serve to protect the adhesive layer and at the same time, efficiently transmit light to the adhesive layer. That is, as the core layer is disposed on one surface of the adhesive layer, it is possible to increase the efficiency with which the adhesive layer transmits light to the site of attachment, and it is possible to improve the durability and the like of the adhesive patch portion.

According to one embodiment of the present disclosure, the photoreactive group-containing compound may include at least one of polyethylene glycol di(meth)acrylate, (meth)acrylated hyaluronic acid, (meth)acrylated alginate, and (meth)acrylated gelatin. Specifically, the photoreactive group-containing compound may include at least one of polyethylene glycol diacrylate and polyethylene glycol dimethacrylate. In addition, the molecular weight of the photoreactive group-containing compound may be 500 g/mol to 3,000 g/mol. By using the above-described type of photoreactive group-containing compound, it is possible to increase the light transmission efficiency of the core layer, and it is possible to produce a core layer having excellent mechanical properties.

According to one embodiment of the present disclosure, as the photoinitiator, any photoinitiator that may be used in the art may be used without limitation. Examples of the photoinitiator include, but are not limited to, a propiophenone-based photoinitiator, a benzoin-based photoinitiator, a benzoin alkyl ether-based photoinitiator, an acetophenone-based photoinitiator, an anthraquinone-based photoinitiator, a thioxanthone-based photoinitiator, a ketal-based photoinitiator, a benzophenone-based photoinitiator, an α-aminoacetophenone-based photoinitiator, an acylphosphine oxide-based photoinitiator, a ketone-based photoinitiator, a phenylphosphine oxide-based photoinitiator, a thioxanthone-based photoinitiator, an oxime ester-based photoinitiator, etc.

According to one embodiment of the present disclosure, the core layer may be produced using a composition for producing a core layer. That is, the core layer may include a photocured product of the composition for producing a core layer. The composition for producing a core layer may contain the photoreactive group-containing compound and the photoinitiator.

According to one embodiment of the present disclosure, the content of the photoreactive group-containing compound may be 5 g to 90 g based on 100 mL of the composition for producing a core layer. That is, the content of the photoreactive group-containing compound may be 5 w/v% to 90 w/v%. When the content of the photoreactive group-containing compound is within the above-described range, it is possible to form a core layer having excellent light transmission efficiency.

According to one embodiment of the present disclosure, the content of the photoinitiator may be 0.01 g to 5 g based on 100 mL of the composition for producing a core layer. That is, the content of the photoinitiator may be 0.01 w/v% to 5 w/v%. When the content of the photoinitiator is within the above-described range, the photocuring reaction for forming the cured product may be stably performed. In addition, by controlling the content of the photoinitiator within the above-described range, it is possible to control the mechanical properties of the core layer, thereby easily producing an adhesive patch portion having desired properties.

According to one embodiment of the present disclosure, the light guide layer may be a hydrogel light-guiding layer including at least one of alginate, hyaluronic acid, chitosan, and gelatin. As the hydrogel light-guiding layer is disposed on the adhesive portion, it may guide light incident from the light-transmitting portion to the adhesive portion. Thereby, the adhesive portion may more stably transmit light to the organic matter surface. In addition, as the light guide layer including the above-described material is disposed on the adhesive portion, a non-uniform or uniform external force applied to the adhesive patch portion may be dispersed, so that the adhesive portion may adhere more stably.

According to one embodiment of the present disclosure, the thickness ratio of the adhesive layer to the core layer may be 1:5 to 1:40. When the thickness ratio of the adhesive layer to the core layer is within the above-described range, the adhesive layer may stably adhere to the organic matter surface, and an external force applied to the adhesive portion may be effectively dispersed.

According to one embodiment of the present disclosure, the thickness ratio of the light guide layer to the adhesive portion may be 1:0.1 to 1:2. That is, the ratio of the thickness of the light guide layer to the sum of the thicknesses of the adhesive layer and the core layer may be 1:0.1 to 1:2. When the thickness ratio of the light guide layer to the adhesive portion is within the above-described range, the adhesive patch portion may effectively transmit light to the organic matter surface. In addition, by controlling the thickness ratio of the light guide layer to the adhesive portion within the above-mentioned range, the light guide layer may disperse an external force, thereby further improving the adhesive stability of the adhesive portion.

According to one embodiment of the present disclosure, the light-transmitting portion may include: a core disposed in contact with one surface of the adhesive portion and configured to guide light to the adhesive patch portion; and a covering layer disposed on the surface of the core. Referring to FIGS. 1a and 1b, one end of the core 210 is disposed in contact with one surface (e.g., a side surface) of the adhesive portion 130, and a light source may be disposed at the other end of the core 210. In addition, the covering layer 220 may be disposed along the outer circumference of the core 210 to cover the entire surface of the core 210. In addition, one end of the covering layer 220 may be disposed in contact with one surface (e.g., a side surface) of the adhesive portion 130 and one surface (e.g., a side surface) of the light guide layer 140, and a light source may be disposed at the other end of the covering layer 220.

According to one embodiment of the present disclosure, the core may serve to primarily transmit light emitted from the light source to the adhesive patch portion. The covering layer may serve to protect the core and as an auxiliary light-transmitting member that transmits light to the adhesive patch portion. That is, by forming the covering layer on the surface of the core, it is possible to increase the light transmission efficiency of the light-transmitting portion and improve the durability and the like of the light-transmitting portion.

According to one embodiment of the present disclosure, the core may include a cured product of a composition containing a photoreactive group-containing compound and a photoinitiator. That is, the core may be a photocured product of a composition for producing a core containing the photoreactive group-containing compound and the photoinitiator.

According to one embodiment of the present disclosure, the photoreactive group-containing compound may include at least one of polyethylene glycol di(meth)acrylate, (meth)acrylated hyaluronic acid, (meth)acrylated alginate, and (meth)acrylated gelatin. Specifically, the photoreactive group-containing compound may include at least one of polyethylene glycol diacrylate and polyethylene glycol dimethacrylate. In addition, the molecular weight of the photoreactive group-containing compound may be 500 g/mol to 3,000 g/mol. By using the above-described type of photoreactive group-containing compound, it is possible to increase the light transmission efficiency of the core, and it is possible to produce a core having excellent mechanical properties.

According to one embodiment of the present disclosure, as the photoinitiator, any photoinitiator that may be used in the art may be used without limitation. Examples of the photoinitiator include, but are not limited to, a propiophenone-based photoinitiator, a benzoin-based photoinitiator, a benzoin alkyl ether-based photoinitiator, an acetophenone-based photoinitiator, an anthraquinone-based photoinitiator, a thioxanthone-based photoinitiator, a ketal-based photoinitiator, a benzophenone-based photoinitiator, an α-aminoacetophenone-based photoinitiator, an acylphosphine oxide-based photoinitiator, a ketone-based photoinitiator, a phenylphosphine oxide-based photoinitiator, a thioxanthone-based photoinitiator, an oxime ester-based photoinitiator, etc.

According to one embodiment of the present disclosure, the core may be produced using a composition for producing a core. That is, the core may include a photocured product of the composition for producing a core. The composition for producing a core may contain the photoreactive group-containing compound and the photoinitiator. The content of the photoreactive group-containing compound may be 5 g to 90 g based on 100 mL of the composition for producing a core. That is, the content of the photoreactive group-containing compound may be 5 w/v% to 90 w/v%. When the content of the photoreactive group-containing compound is within the above-described range, it is possible to form a core having excellent light transmission efficiency.

According to one embodiment of the present disclosure, the content of the photoinitiator may be 0.01 g to 5 g based on 100 mL of the composition for producing a core. That is, the content of the photoinitiator may be 0.01 w/v% to 5 w/v%. When the content of the photoinitiator in the composition for producing a core is within the above-described range, the photocuring reaction for forming the cured product may be stably performed. In addition, by controlling the content of the photoinitiator within the above-described range, it is possible to control the mechanical properties of the core, making the light-transmitting portion flexible and bendable.

According to one embodiment of the present disclosure, the covering layer may include at least one hydrogel layer including at least one of alginate, hyaluronic acid, chitosan, and gelatin. Specifically, the covering layer may include at least one hydrogel layer including at least one of the above-mentioned materials, and two or more hydrogel layers may be formed of the same material or may be formed of different materials. In addition, the two or more hydrogel layers included in the covering layer may have the same or different thicknesses. As the covering layer including at least one of the above-mentioned materials is disposed on the core, it may assist in the transmission of light through the core and prevent the core from being damaged.

According to one embodiment of the present disclosure, the thickness ratio of the core to the covering layer may be 1:0.2 to 1:4. When the thickness ratio of the core to the covering layer is within the above range, the light-transmitting portion may effectively transmit light to the adhesive patch portion. In addition, the light-transmitting portion may be prevented from being damaged, and the light-transmitting portion may be easily deformed. That is, the light-transmitting portion may be bent without being damaged, thereby improving the usability of the light-guiding adhesive patch.

According to one embodiment of the present disclosure, the ratio of the area of the adhesive portion to the cross-sectional area of the core may be 1:0.004 to 1:0.04. That is, the ratio of the area of the adhesive layer to the cross-sectional area of the core may be 1:0.004 to 1:0.04, and the ratio of the area of the core layer to the cross-sectional area of the core may be 1:0.004 to 1:0.04. Referring to FIG. 1a, the area of the adhesive portion may refer to the cross-sectional area of the adhesive portion in the x-y plane, and the cross-sectional area of the core may refer to the cross-sectional area of the core in the y-z plane. In this case, light may be irradiated to the core in the x-axis direction (the direction perpendicular to the y-z plane). When the ratio of the area of the adhesive portion to the cross-sectional area of the core is within the above-described range, light emitted from the light source may stably reach the adhesive patch portion through the light-transmitting portion.

One embodiment of the present disclosure provides a method for producing a light-guiding adhesive patch, including steps of: producing an adhesive patch portion; and producing a light-transmitting portion.

According to the method for producing a light-guiding adhesive patch according to one embodiment of the present disclosure, it is possible to easily produce a light-guiding adhesive patch that may stably adhere to the organic matter surface and effectively transmit light to the site of attachment.

The method for producing a light-guiding adhesive patch may be a method for producing the light-guiding adhesive patch according to the above-described embodiment. In the method for producing a light-guiding adhesive patch according to this embodiment, the adhesive layer, the core layer, the adhesive portion, the light guide layer, the adhesive patch portion, the core, the covering layer, and the light-transmitting portion may be the same as the adhesive layer, core layer, adhesive portion, light guide layer, adhesive patch portion, core, covering layer, and light-transmitting portion included in the light-guiding adhesive patch according to the above-described embodiment.

According to one embodiment of the present disclosure, the step of producing the adhesive patch portion may include steps of: producing an adhesive layer; producing a core layer; and producing a light guide layer.

The step of producing the adhesive layer may include producing the adhesive layer using a composition for producing an adhesive layer containing the biocompatible polymer and the pyrogallol-based compound. Specifically, this step may include drying the composition for producing an adhesive layer at a temperature of 20°C to 40°C to obtain a film-shaped adhesive layer, and shaping the obtained adhesive layer into a desired shape.

The step of producing the core layer may include producing the core layer using a composition for producing a core layer containing the photoreactive group-containing compound and the photoinitiator. Specifically, this step may include placing the composition for producing a core layer in a mold, and curing the composition by irradiation with UV light at an intensity of 1 mW/cm² to 100 mW/cm² for 5 minutes to 10 minutes, thereby producing the core layer.

The adhesive portion may be produced by attaching an adhesive layer onto the produced core layer. In this case, the core layer may be attached to one surface of the adhesive layer, and a release film may be attached to the other surface of the adhesive layer, which is exposed to the outside.

The step of producing the light guide layer may include producing the light guide layer using a hydrogel precursor solution. Here, the hydrogel precursor solution may include at least one of alginate, hyaluronic acid, chitosan, and gelatin. Specifically, this step may include applying the hydrogel precursor solution to one surface of the core layer of the adhesive portion and immersing the resulting structure in a reactive solution to form the light guide layer on the core layer. Here, the reactive solution may include a reactive material capable of reacting with a compound contained in the hydrogel precursor solution to form a hydrogel, wherein the reactive material may be, for example, calcium chloride.

According to one embodiment of the present disclosure, the step of producing the light-transmitting portion may include steps of: producing a core; and producing a covering layer.

The step of producing the core may include producing the core using the composition for producing a core including the photoreactive group-containing compound and the photoinitiator. Specifically, this step may include placing the composition for producing a core in a mold, and curing the composition for producing a core by irradiation with UV light at an intensity of 1 mW/cm² to 100 mW/cm² for 5 minutes to 10 minutes, thereby producing the core.

The step of producing the covering layer may include producing the covering layer using a hydrogel precursor solution. Here, the hydrogel precursor solution may include at least one of alginate, hyaluronic acid, chitosan, and gelatin. Specifically, this step may include immersing the produced core in the hydrogel precursor solution, followed by immersion in a reactive solution to form the covering layer on the core. Here, the reactive solution may include a reactive material capable of reacting with a compound contained in the hydrogel precursor solution to form a hydrogel, wherein the reactive material may be, for example, calcium chloride.

According to one embodiment of the present disclosure, the light-guiding adhesive patch may be produced by attaching the produced light-transmitting portion to one side of the produced adhesive patch portion. To attach the light-transmitting portion to the adhesive patch portion, an adhesive or adhesive film harmless to the human body used in the art may be used. Alternatively, the light-guiding adhesive patch may be produced by positioning the light-transmitting portion on one side of the adhesive patch portion, and bonding the light-transmitting portion to the adhesive patch portion using the hydrogel precursor solution and the reactive solution. Alternatively, the produced core may be positioned on one side of the adhesive portion including the adhesive layer and the core layer, and the light guide layer and the covering layer may be formed together using the hydrogel precursor solution and the reactive solution. Thereby, the light-guiding adhesive patch including the adhesive patch portion integral with the light-transmitting portion may be produced.

Hereinafter, the present disclosure will be described in detail by way of examples. However, the examples according to the present disclosure may be modified into various different forms, and the scope of the present disclosure is not interpreted as being limited to the examples described below. The examples of the present specification are provided to more completely explain the present disclosure to those skilled in the art.

### Examples

### Example 1

A light-guiding adhesive patch having the configuration shown in FIG. 1a was produced using the following method.

### 1) Production of Adhesive Patch Portion

### (1) Production of Core Layer

A composition for producing a core layer was prepared by mixing a polyethylene glycol diacrylate having a molecular weight of 700 g/mol, which is a photoreactive group-containing compound, and 2-hydroxy-4'-(2-hydroxyethoxy)-2-methylpropiophenone (Sigma Aldrich) as a photoinitiator in distilled water.

Here, the content of the polyethylene glycol diacrylate was 80 g (80 w/v%) based on 100 mL of the composition for producing a core layer. In addition, the content of the photoinitiator was 1 g (1 w/v%) based on 100 mL of the composition for producing a core layer.

Thereafter, the composition for producing a core layer was placed in a mold and irradiated with UV light at an intensity of 5 mW/cm² for 300 seconds. Finally, a core layer having a width of 15 mm, a length of 15 mm, and a thickness of 2 mm was produced.

### (2) Production of Adhesive Layer

Hyaluronic acid-adipic acid dihydrazide as an amine group-containing biocompatible polymer and 2,3,4-trihydroxybenzaldehyde (2,3,4-trihydroxybenzaldehyde) as a pyrogallol compound were prepared.

Afterwards, a composition for preparing an adhesive layer was prepared by mixing hyaluronic acid-adipic acid dihydrazide, 2,3,4-trihydroxybenzaldehyde, and distilled water. In this case, the content of hyaluronic acid-adipic acid dihydrazide was 1 part by weight based on 100 parts by weight of the composition for preparing an adhesive layer. Meanwhile, the amount of 2,3,4-trihydroxybenzaldehyde was adjusted so that the amine group of the 6-arm polyethylene glycol amine and the aldehyde group of 2,3,4-trihydroxybenzaldehyde corresponded to 1:0, 1:1, and 1:5. In this case, the content of 2,3,4-trihydroxybenzaldehyde was 0, 10, and 50 parts by weight, respectively, with respect to 100 parts by weight of the total content of hyaluronic acid-adipic acid dihydrazide

Thereafter, the composition for producing an adhesive layer was dried at room temperature to obtain a film-shaped adhesive layer which was then shaped, thereby producing an adhesive layer having a width of 15 mm, a length of 15 mm, and a thickness of 0.1 mm. Then, the produced adhesive layer was attached to one surface of the core layer produced as described above, thereby producing an adhesive portion.

### (3) Production of Light Guide Layer

A 2 w/v% alginate solution and a 100 mM calcium chloride solution were prepared.

Thereafter, the alginate solution was applied to the other surface of the core layer to which the adhesive layer was not attached, and then the resulting structure was immersed in the calcium chloride solution to form an alginate hydrogel. That is, a light guide layer including the alginate hydrogel was formed on the other side of the core layer. Here, the light guide layer formed on the core layer was 15 mm in width, 15 mm in length, and 1 mm in thickness. Thereby, an adhesive patch portion was finally produced.

### 2) Production of Light-Transmitting Portion

### (1) Production of Core

### Example 1-1 (Photoinitiator: 0.2 w/v%)

A composition for producing a core was prepared by mixing a polyethylene glycol diacrylate having a molecular weight of 700 g/mol, which is a photoreactive group-containing compound, and 2-hydroxy-4'-(2-hydroxyethoxy)-2-methylpropiophenone (Sigma Aldrich) as a photoinitiator in distilled water. Here, the content of the polyethylene glycol diacrylate was 80 g (80 w/v%) based on 100 mL of the composition for producing a core. In addition, the content of the photoinitiator was 0.2 g (0.2 w/v%) based on 100 mL of the composition for producing a core.

Thereafter, the composition for producing a core was placed in a mold and irradiated with UV light at an intensity of 5 mW/cm² for 300 seconds. Finally, a core having a diameter of 2 mm and a length of 30 mm was produced.

### Example 1-2 (Photoinitiator: 0.6 w/v%)

A light-transmitting core was produced in the same manner as in Example 1-1, except that the content of the photoinitiator was adjusted to 0.6 w/v%.

### Example 1-3 (Photoinitiator: 1 w/v%)

A light-transmitting core was produced in the same manner as in Example 1-1, except that the content of the photoinitiator was adjusted to 1 w/v%.

### Example 1-4 (Photoinitiator: 1 w/v%, and UV intensity: 50 mW/cm²)

A light-transmitting core was produced in the same manner as in Example 1-1, except that the content of the photoinitiator was adjusted to 1 w/v% and the UV intensity was adjusted to 50 mW/cm².

### (2) Production of Covering Layer

A 2 w/v% alginate solution and a 100 mM calcium chloride solution were prepared.

Thereafter, the produced core was immersed in the alginate solution, and then immersed in the calcium chloride solution to form an alginate hydrogel. That is, a covering layer including the alginate hydrogel was formed on the surface of the core. Here, the covering layer had a thickness of 0.5 mm and a length of 30 mm. Thereby, a light-transmitting portion was finally produced.

Thereafter, a light-guiding adhesive patch having the light-transmitting portion connected to one side of the adhesive patch portion was produced.

### Example 2

A light-guiding adhesive patch having the configuration shown in FIG. 1b was produced using the following method.

### 1) Production of Adhesive Patch Portion

### (1) Production of Core Layer

A core layer having a diameter of 8 mm and a thickness of 2 mm was produced in the same manner as in Example 1.

### (2) Production of Adhesive Layer

An adhesive layer having a diameter of 8 mm and a thickness of 0.1 mm was produced in the same manner as in Example 1. Then, the produced adhesive layer was attached to one surface of the produced core layer, thereby producing an adhesive portion.

### (3) Production of Light Guide Layer

Using the same method as in Example 1, a light guide layer having a diameter of 8 mm and a thickness of 1 mm was formed on the other surface of the core layer. Thereby, an adhesive patch portion was finally produced.

### 2) Production of Light-Transmitting Portion

### (1) Production of Core

A light-transmitting core having a diameter of 2 mm and a length of 30 mm was produced in the same manner as in Example 1.

### (2) Production of Covering Layer

Using the same method as in Example 1, a covering layer having a thickness of 0.5 mm was produced on the surface of the produced core. Thereby, a light-transmitting portion was finally produced.

Thereafter, a light-guiding adhesive patch having the light-transmitting portion connected to one side of the adhesive patch portion was produced.

### Experimental Examples

### Measurement of Mechanical Properties of Light-Transmitting Cores

The mechanical properties of the light-transmitting core produced in each of Examples 1-1 to 1-4 were measured using a Discovery Hybrid Rheometer 2 (TA Instruments). Specifically, the storage modulus (G'), loss modulus (G"), and Tan (δ) values of the light-transmitting cores were measured at a frequency of 1 Hz.

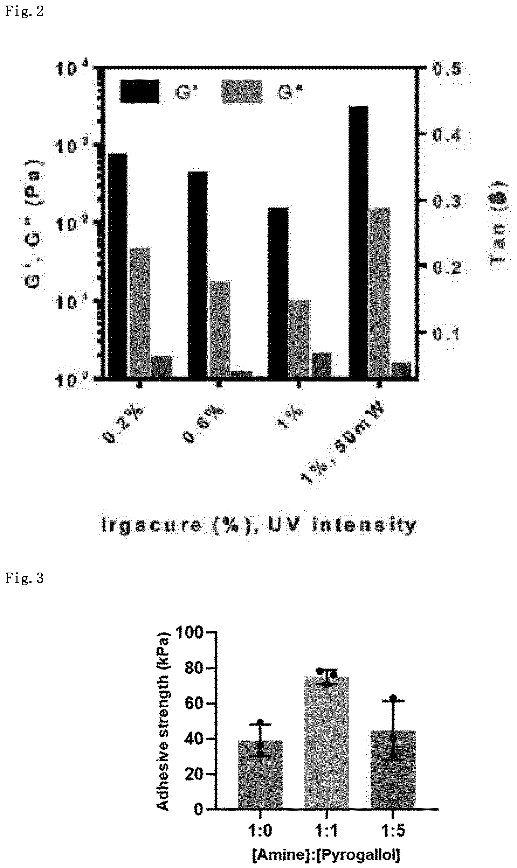

FIG. 2 shows the results of measuring the mechanical properties of the light-transmitting cores produced in Example 1 of the present disclosure. Specifically, FIG. 2 shows the measured storage modulus (G'), loss modulus (G"), and Tan (δ) values of the light transmitting cores produced in Examples 1-1 to 1-4.

Referring to FIG. 2, it can be seen that the mechanical properties of the core produced may be controlled by adjusting the content of the photoinitiator in the composition for producing a core.

### Ex vivo adhesion evaluation of adhesive layer

Using a universal testing machine (UTM; 34SC-1, Instron), the adhesive force of the adhesive layer prepared by the same method as Example 1 was measured. Specifically, PET film was attached as a backing substrate to pig skin tissue that was first processed to 1 cm × 2 cm. The adhesive layer was molded to be 1 cm × 1 cm and attached between two pig skin tissues, and was pulled at a speed of 20 mm/min after 5 minutes, and the attachment strength (kPa) was calculated as the maximum load (N) compared to the area to which the film was attached.

FIG. 3 is a result of measuring the adhesive force of the adhesive layer prepared in Example 1 of the present invention. Specifically, the adhesive force (kPa) of the adhesive layer prepared in Example 1 is shown.

Referring to FIG. 3, it may be seen that the adhesive force of the adhesive layer may be controlled by adjusting the content of 2,3,4-trihydroxybenzaldehyde, which is a pyrogallol-based compound.

### Evaluation of Light Transmission

It was evaluated whether the light-transmitting portions produced in the Examples would transmit light incident from the outside well.

Specifically, Avalight-LED (Avantes) was prepared as a light irradiation device capable of controlling the wavelength of light. One end of the light-transmitting portion produced in each of Examples 1 and 2 was connected to the light irradiation device. Then, the light irradiation device was driven and it was examined whether the light-transmitting portion could transmit light.

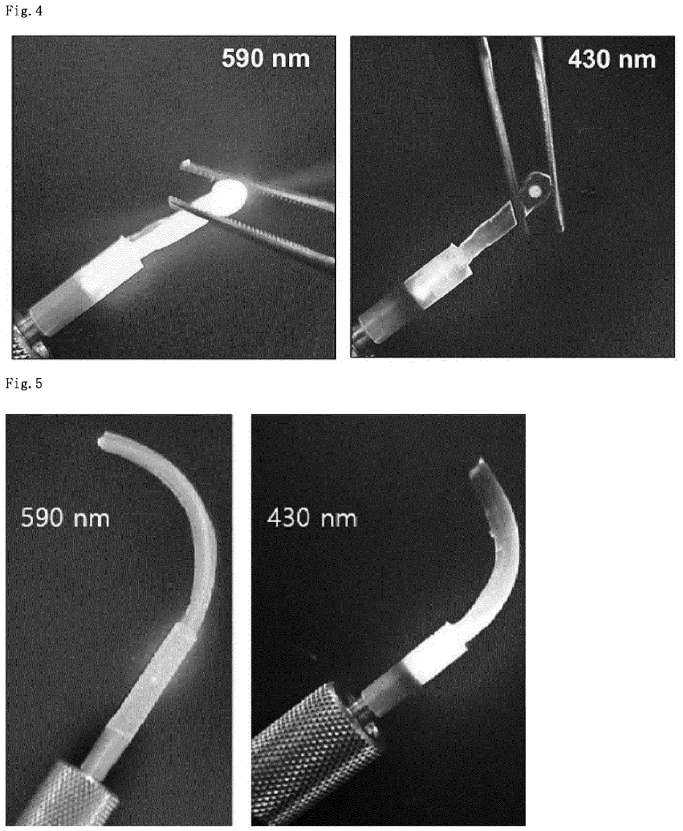

FIG. 4 depicts photographs showing that the light-transmitting portion produced in Example 1 of the present disclosure transmits light. FIG. 5 depicts photographs showing that a light-transmitting portion produced in Example 2 of the present disclosure transmits light.

Referring to FIGS. 4 and 5, it was confirmed that the light transmitting portion produced in each of Examples 1 and 2 transmitted incident light well from one end to the other end. In particular, referring to FIG. 5, it was confirmed that, even when the light-transmitting portion was bent, it transmitted incident light well from one end to the other end.

In addition, it was evaluated whether the light-guiding adhesive patches produced in the Examples would transmit light incident from the outside well.

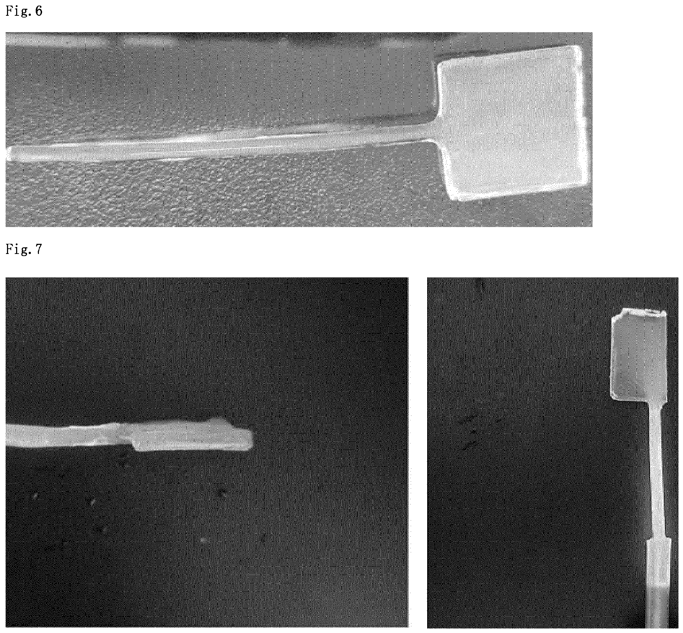

FIG. 6 is a photograph of the light-guiding adhesive patch produced in Example 1 of the present disclosure, and

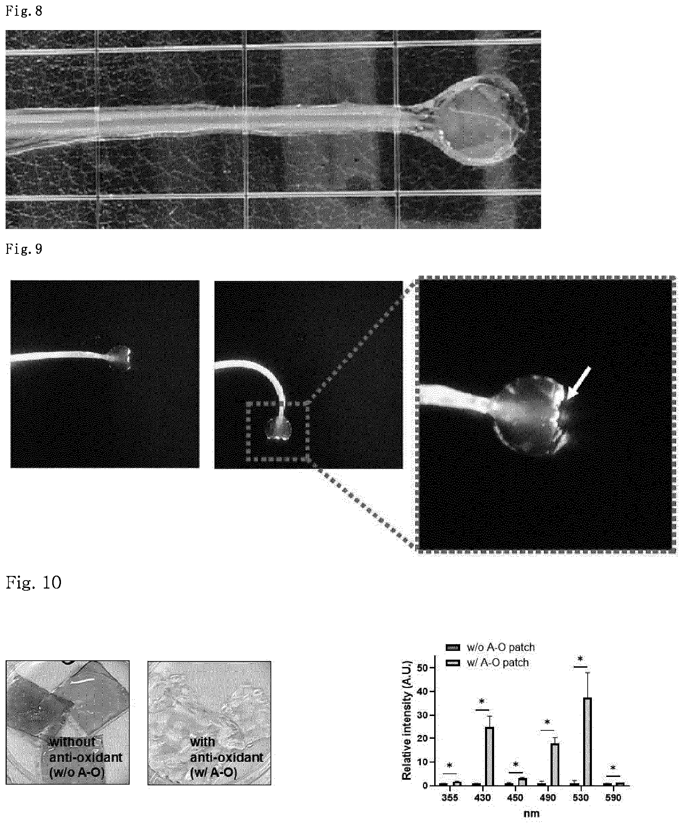

FIG. 7 depicts photographs showing that the light-guiding adhesive patch produced in Example 1 of the present disclosure transmits light. FIG. 8 is a photograph of the light-guiding adhesive patch produced in Example 2 of the present disclosure, and FIG. 9 depicts photographs showing that the light-guiding adhesive patch produced in Example 2 of the present disclosure transmits light.

Referring to FIGS. 7 and 9, it was confirmed that the light-guiding adhesive patch produced in each of Examples 1 and 2 transmitted incident light well from the end of the light-transmitting portion to the adhesive patch portion. In particular, referring to FIG. 9, it was confirmed that, even when the light-transmitting portion was bent, the light-guiding adhesive patch transmitted incident light well from the end of the light-transmitting portion to the adhesive patch portion.

### Improvement in Light Transmission Rate

To improve the light transmission rate of the existing brown-colored bio-adhesive patch (without anti-oxidant, w/o A-O) of Examples 1 and 2, transparency was achieved using an anti-oxidant (A-O) in a newly developed light-guiding adhesive patch. The light transmission rate was measured under the patch using LED within the light wavelength range of 355 nm to 590 nm for photodynamic therapy (PDT) employing the Hypericin photosensitizer. Results shown in Fig. 10, although there were variations depending on the light wavelength, the developed 'w/ A-O' patch group achieved statistically significant improvements in light transmission rates, ranging from a minimum of 1.2 times to a maximum of 36.3 times, compared to the 'w/o A-O' group (Mann-Whitney nonparametric unpaired t-test; *: p-value < 0.05).

### Example of Photodynamic Therapy (PDT) Implementation

To maximize light transmission, a 530 ± 15 nm LED was used to verify the improvement in PDT efficiency with a developed light-guiding adhesive patch using the malignant glioma U87 cell line.

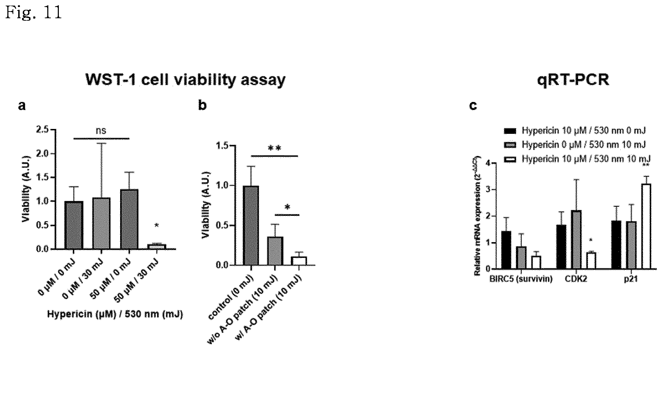

Specifically, the anti-cancer effectiveness through PDT was verified using the WST-1 cell viability assay. During the PDT efficiency verification, initial tests were conducted to establish PDT conditions, investigating the toxicity of the photosensitizer and light exposure. No specific inhibition of cell viability was observed with a 50 µM Hypericin photosensitizer concentration (2 hours incubation at 37°C) and within the 530 nm 30 mJ light exposure range. However, statistically significant cell death (reduction in cell viability) was observed when the photosensitizer and light exposure were applied simultaneously (Fig. 11a). Subsequent comparison of PDT efficiency using 'w/o A-O' and 'w/ A-O' patches demonstrated a statistically significant improvement in PDT-induced cell death efficiency with the 'w/ A-O' patch (Fig.11b). Changes in cell signaling related to cell cycle markers CDK2 and p21, measured by qRT-PCR, indicated significant alterations when PDT was performed using the 'w/ A-O' patch, affecting the cell death mechanism as well (Fig. 11c).

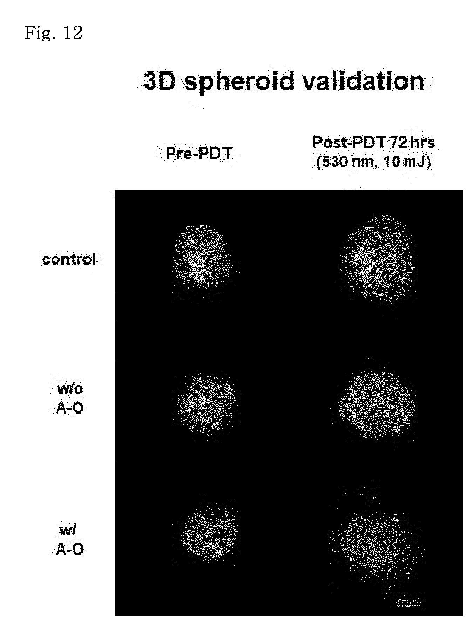

Additionally, compared to 2D cell cultures, verification using 3D spheroids, which are closer to in vivo conditions, also showed that the 'w/ A-O' patch group led to cell growth inhibition and induced cell death-necrosis at the periphery, confirming the improvement in PDT efficiency with the developed 'w/ A-O' light-guiding adhesive patch (Fig. 12) .

## Claims

1. A light-guiding adhesive patch comprising:
an adhesive patch portion comprising an adhesive portion comprising an adhesive layer and a core layer, and a light guide layer disposed on the adhesive portion and configured to guide light to the adhesive portion; and
a light-transmitting portion connected to the adhesive patch portion and configured to guide light incident from an outside to the adhesive patch portion.

2. The light-guiding adhesive patch of claim 1, wherein the adhesive layer is a hydrogel adhesive layer comprising a reaction product of a biocompatible polymer and a pyrogallol-based compound.

3. The light-guiding adhesive patch of claim 2, wherein the biocompatible polymer comprises at least one of polyethylene glycol, alginate, hyaluronic acid, hyaluronic acid-adipic acid dihydrazide, gelatin, polyethylene glycol amine, 4-arm polyethylene glycol amine, 6-arm polyethylene glycol amine, 8-arm polyethylene glycol amine, chitosan, and collagen.

4. The light-guiding adhesive patch of claims 2 or 3, wherein the pyrogallol-based compound is a compound represented by Formula 1 below: 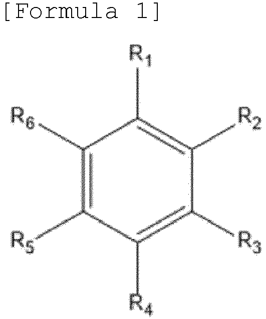
wherein R₁ is -COOH, -CHO, -NH₂, -SH, a straight or branched chain alkyl having 1 to 10 carbon atoms, or a straight or branched chain alkenyl having 2 to 10 carbon atoms; and
three of R₂ to R₆ are -OH and the remaining two are hydrogen atoms.

5. The light-guiding adhesive patch of claims 2-4, wherein the reaction product contains the pyrogallol-based compound in an amount of 0.3 parts by weight to 30 parts by weight based on 100 parts by weight of the biocompatible polymer.

6. The light-guiding adhesive patch of claims 1-5, wherein the core layer is a hydrogel comprising a cured product of a composition containing a photoreactive group-containing compound and a photoinitiator.

7. The light-guiding adhesive patch of claim 6, wherein the photoreactive group-containing compound comprises at least one of polyethylene glycol di(meth)acrylate, (meth)acrylated hyaluronic acid, (meth)acrylated alginate, and (meth)acrylated gelatin.

8. The light-guiding adhesive patch of claims 1-7, wherein the light guide layer is a hydrogel light-guiding layer comprising at least one of alginate, hyaluronic acid, chitosan, and gelatin.

9. The light-guiding adhesive patch of claims 1-8, wherein a thickness ratio of the light guide layer to the adhesive portion is 1:0.1 to 1:2.

10. The light-guiding adhesive patch of claim 1-9, wherein the light-transmitting portion comprises:
a core disposed in contact with one surface of the adhesive layer and configured to guide light to the adhesive patch portion; and
a covering layer disposed on the surface of the core.

11. The light-guiding adhesive patch of claim 10, wherein the core is a hydrogel comprising a cured product of a composition containing a photoreactive group-containing compound and a photoinitiator.

12. The light-guiding adhesive patch of claim 11, wherein the photoreactive group-containing compound comprises at least one of polyethylene glycol di(meth)acrylate, (meth)acrylated hyaluronic acid, (meth)acrylated alginate, and (meth)acrylated gelatin.

13. The light-guiding adhesive patch of claims 10-12, wherein the covering layer comprises at least one hydrogel layer including at least one of alginate, hyaluronic acid, chitosan, and gelatin.

14. The light-guiding adhesive patch of claims 10-13, wherein a thickness ratio of the core to the covering layer is 1:0.2 to 1:4.

15. The light-guiding adhesive patch of claims 1-14, wherein a ratio of an area of the adhesive portion to a cross-sectional area of the core is 1:0.004 to 1:0.04.
